# EUROPEAN PATENT SPECIFICATION

(11) **EP 4 586 813 B1**
(45) Date of publication and mention of the grant of the patent: **27.05.2026**
(21) Application number: 23762215.4
(22) Date of filing: 28.08.2023
(51) Int. Cl.: A01K 67/36, A01K 67/364

(54) **AN INSECT REARING/BREEDING HOUSING**
INSEKTENZUCHTGEHÄUSE
ENCEINTE POUR L'ÉLEVAGE D'INSECTES

(30) Priority: 14.09.2022 DK PA202270443
(43) Date of publication of application: 23.07.2025
(73) Proprietor: SKOV A/S, Glyngoere, 7870 Roslev (DK)
(72) Inventor: HECKMANN, Lars-Henrik Lau, 7870 Roslev (DK); SCHOU, Toke Munk, 8762 Flemming (DK); CHRISTENSEN, Michael Hyldig, 7870 Roslev (DK); LAURIDSEN, Arne Blok, 8762 Flemming (DK); VOIGT, Henrik, 7870 Roslev (DK); PEDERSEN, Carsten Lind, 8762 Flemming (DK)
(74) Representative: Zacco Denmark A/S
(86) International application number: PCT/EP2023/073513
(87) International publication number: WO 2024/056355

(56) References cited:
- WO-A1-2014/171829
- WO-A1-2022/024151
- CN-Y- 2 777 970
- KR-A- 20210 156 940
- KR-A- 20220 037 893

## Description

### Technical field

The present disclosure relates to an insect rearing/breeding housing.

### Background art

In known insect rearing/breeding housings for example as mentioned in WO2020245158 A1 a number of crates are placed in a rearing/breeding housing in stacks. A problem with the solutions of the prior art is that providing proper and uniform ventilation or conditioned air to each crate in a housing for rearing insect larvae is a challenge and needs dedicated nozzles for the individual crates placed in the housing. There is thus a need for improved equipment for rearing/breeding insects.

KR 2022 0037893 A, CN 2 777 970 Y, WO 2022/024151 A1 and KR 2021 0156940 A disclose examples of rearing/breeding devices.

### Summary

It is an object of the present disclosure to mitigate, alleviate or eliminate one or more of the above-identified deficiencies and disadvantages in the prior art and solve at least the above-mentioned problem. According to a first aspect, there is provided an insect rearing/breeding housing, the housing being defined by one end, an opposite end, sides and a top, wherein a plurality of crates having a bottom and an upper edge and arranged in a row along the length of the housing define at least a portion of the housing bottom, and wherein holes formed in the bottom of the crates allow for air to be sucked into the housing and to be discharged through at least one air ventilating opening of the housing, the housing having a crate insertion opening in at least one end and a crate discharge opening at the housing bottom, the crates being supported so as to be movable from the insertion opening to the discharge opening.

Hereby it is possible to provide an even ventilation or air stream through the crates and the housing.

According to some embodiments, the crate discharge opening is provided at an opposite end in relation to a crate insertion opening at one end and at the housing bottom,

Hereby is achieved that crates can be inserted into the rearing/breeding housing in one end of the housing and can be removed from the opposite end of the housing.

According to some embodiments, the housing is provided with a crate insertion opening at both ends of said housing and a discharge opening is provided at a middle portion of the housing at the housing bottom.

Hereby is achieved that crates can be inserted in both ends of the housing and that crates can be removed from a middle part of the housing.

According to some embodiments, longitudinal support flanges for supporting the crates is provided at each side of the housing extending from the crate insertion opening to the crate discharge opening.

Hereby is achieved a connection between the crates and the housing where the crates can be moved through the housing from the crate insertion opening in at least one end and towards the crate discharge opening and at the same time provide a connection preventing the flying insects from escaping the housing since the flanges and the crates forms a connection where an edge of the crate slide on an upper side of the flanges at each side of the housing.

According to some embodiments, a longitudinal flange is provided at each side of the housing, parallel to the support flanges and having a distance from the support flanges corresponding to a height of an edge of the crates in such a way that the edge of the crates can be moved along the support flanges.

According to some embodiments, the longitudinal support flanges for supporting the crates provided at each side of the housing, extends from the crate insertion openings at each end of the housing to the crate discharge opening provided at a middle portion of the housing at the housing bottom.

Hereby is achieved that the edges of crates abutting each other when moved from the crate insertion opening to the crate discharge opening, are aligned and do not tend to be displaced in height in such a way that the edges will form an air gap in one end relative to the flanges.

The further longitudinal flanges are preferably placed above the support flanges.

According to some embodiments, the longitudinal support flanges leaves a gap at each side of the housing, for removal of the crates.

Hereby it is possible to remove crates by lowering the crates and thereby remove crates from the housing bottom.

According to some embodiments, a cover plate is provided above and between the gaps, which cover plate slopes downwards to at least one side along a moving direction of the crates.

Hereby is achieved that dead flies or the like will fall into the crates and thereby removed by the discharged crates to avoid dead flies or the like generating smells attracting flies to lay eggs different places than in the egg module.

According to some embodiments, a separating wall or plate extends from a top part of the cover plate to the top of the housing, separating the housing in two.

This makes it possible for rearing/breeding in two different populations in the same housing, still keeping the populations separated from each other.

According to some embodiments, the cover plate is provided with a sealing for sealing towards an upper edge of the crates.

Hereby is achieved that insects are prevented from escaping the housing when discharging crates from the discharge opening.

According to some embodiments, ventilating air is supplied through the bottom of the housing from below the crates.

This is an advantage since it is possible to have a housing with an open bottom structure, where the bottom opening is closed by crates with a plurality of perforations in the bottom of the crates, which crates are used for housing the pupae. Hereby it is also possible to remove smells from i.e. pupae remains or the like, from the housing.

According to some embodiments, ventilating air is sucked out at top of the housing by a fan or blower.

Hereby it is possible to suck out air from the housing or to assist removing air by blowers, fans or the like, which air may be forced into the housing by blowers, fans or the like or just sucked into the housing through the bottom and out through top openings.

According to some embodiments, ventilating air is supplied through one or more openings at a top part of the housing.

Hereby is disclosed an alternative way of supplying ventilating air to the housing.

To further enhance ventilating flow through the crates, a kind of curtains are provided extending along sides of the crates from the longitudinal support flanges and to at least an underside of the crates. This ensures a directional flow of air, which is thereby forced or guided to primarily go up through the bottom of the crates and not partially through the holes in the sides of the crates.

The curtains can be made of metal, plastic or another suitable substantially rigid material that will not flap under the influence of the ventilation air.

In a simple embodiment, the curtains are formed by the sides of the housing, which sides continues from the top of the housing to below or to a point level with the underside of the crates when placed in the housing.

According to some embodiments, ventilating air is alternating between being supplied through one or more openings at a top part of the housing and through the bottom from below the crates.

Hereby is disclosed yet an alternative way of supplying ventilating air to the housing. Alternating the air stream may clear openings or perforations in the bottom of the crates when covered by remains from the pupae.

According to some embodiments, one or more ventilating openings at top of the housing are provided with a net or grate, preventing flies from entering the one or more ventilating openings.

Hereby is prevented that flying insects, for example flies can escape the housing by entering the one or more ventilating openings.

According to some embodiments, an inspection window is provided in at least one end of the housing.

Hereby it is possible to follow the course of the rearing/breeding process to be able to intervene if necessary.

According to some embodiments, crate insertion openings and crate discharge openings are provided with a sealing for sealing towards an upper edge of the crates.

Hereby is also achieved that insects are prevented from escaping the housing when discharging crates from the discharge opening.

According to some embodiments, a conveyor is provided at the discharge opening for conveying discharged crates in a crosswise direction in relation to movement of the crates in the housing.

According to some embodiments, the conveyor is placed below the crate discharge opening at a middle portion of the housing at the housing bottom.

Providing a conveyor to handle discharged crates from the housing gives possibilities for building of a more flexible rearing/breeding system comprising a plurality of rearing/breeding housings placed next to each other.

The present disclosure will become apparent from the detailed description given below. The detailed description and specific examples disclose preferred embodiments of the disclosure by way of illustration only. Those skilled in the art understand from guidance in the detailed description that changes and modifications may be made within the scope of the disclosure.

Hence, it is to be understood that the herein disclosed disclosure is not limited to the particular component parts of the device described or steps of the methods described since such device and method may vary. It is also to be understood that the terminology used herein is for purpose of describing particular embodiments only, and is not intended to be limiting. It should be noted that, as used in the specification and the appended claim, the articles "a", "an", "the", and "said" are intended to mean that there are one or more of the elements unless the context explicitly dictates otherwise. Thus, for example, reference to "a unit" or "the unit" may include several devices, and the like. Furthermore, the words "comprising", "including", "containing" and similar wordings does not exclude other elements or steps.

### Brief descriptions of the drawings

The above objects, as well as additional objects, features and advantages of the present disclosure, will be more fully appreciated by reference to the following illustrative and non-limiting detailed description of example embodiments of the present disclosure, when taken in conjunction with the accompanying drawings.
Figure 1 shows a rearing/breeding housing where a row of crates is inserted in the bottom according to an embodiment of the present disclosure;
Figure 2 shows an empty rearing/breeding housing. according to an embodiment of the present disclosure;
Figure 3 shows a front view of a rearing/breeding housing where a row of crates is inserted in the bottom according to an embodiment of the present disclosure;
Figure 4 shows an empty rearing/breeding housing seen from the insertion end from where crates can be inserted according to an embodiment of the present disclosure;
Figure 5 shows schematically a possible ventilating flow in a rearing/breeding housing according to the embodiment in Figure 1;
Figure 6 shows a rearing/breeding housing where a row of crates is inserted in the bottom according to an alternative embodiment of the present disclosure;
Figure 7 shows the embodiment of figure 6 where a side is removed;
Figure 8 shows an embodiment where the housing is separated in two rearing/breeding spaces;
Figure 9 shows schematically an alternative embodiment provided with a conveyor for transport of discharged crates; and
Figure 10 shows schematically a facility comprising a plurality of rearing/breeding housings.

### Detailed description

The present disclosure will now be described with reference to the accompanying drawings, in which preferred example embodiments of the disclosure are shown. The disclosure may, however, be embodied in other forms and should not be construed as limited to the herein disclosed embodiments. The disclosed embodiments are provided to fully convey the scope of the disclosure to the skilled person.

The insect rearing/breeding housing 1 can be used for rearing/breeding of various kinds of flying insects.

In the following an insect rearing/breeding housing 1 for Black Soldier Fly hereafter called BSF, but not limited hereto, will be described.

When BSF larvae mature, they grow into approx. 12 mm long grubs, a point (after approximately 2-3 weeks) at which they become pre-pupa and finally turn into pupae at which condition they may be up to 25 - 30 mm long. The BSF larvae can be fed to for example fish, poultry and pigs and are a good source of protein. It is also possible to dry the larvae to be processed into feed for use at a later time.

Following minimum one week of maturation, the pupae emerge as flies that breed, propagating the population.

In this way it is possible to produce enough flies for maintaining the population as well as growing larvae to provide feed to i.e. chickens or other live animals.

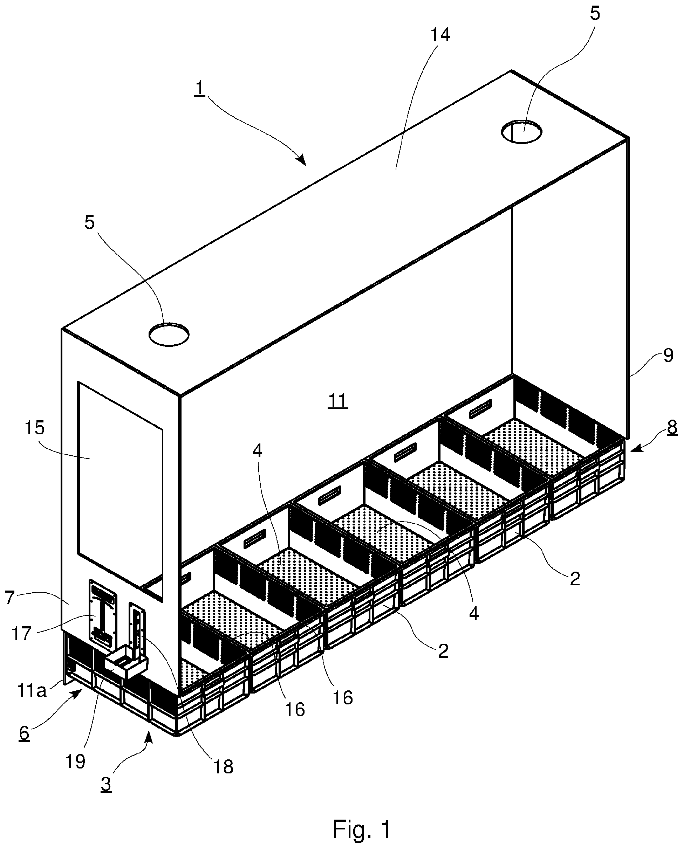

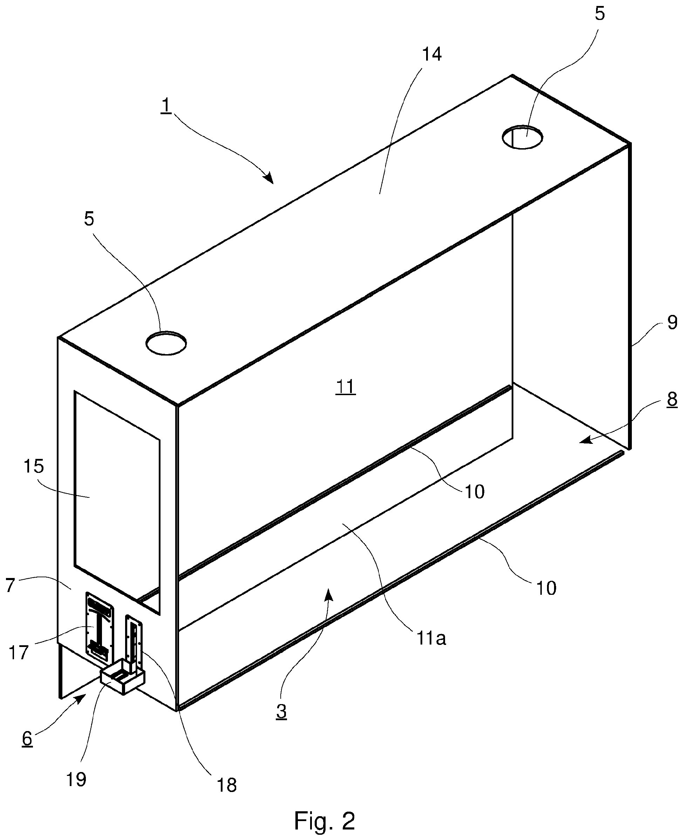

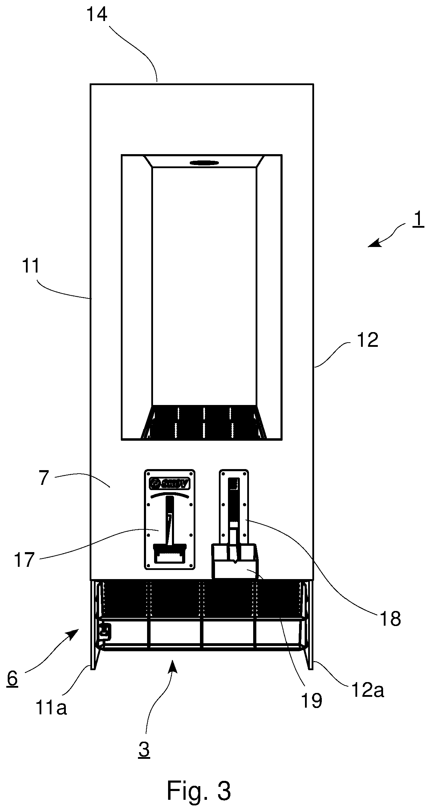

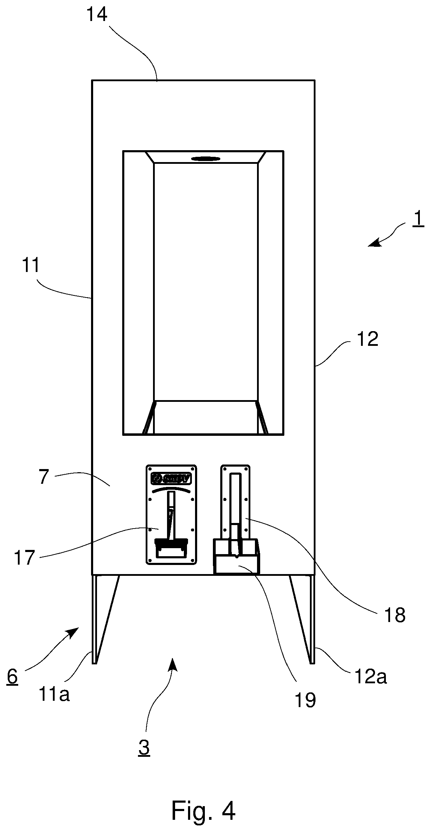

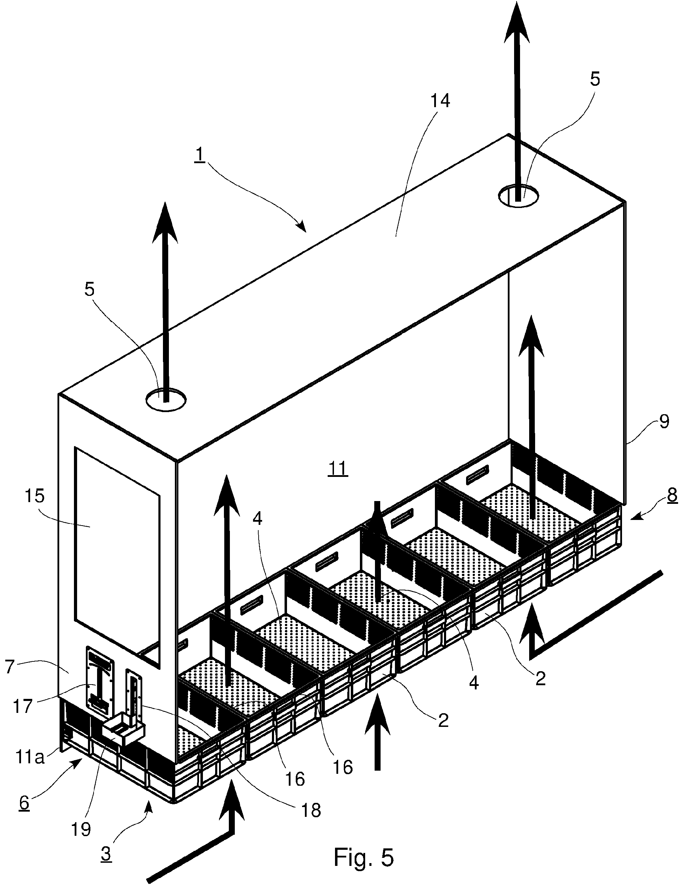

Figure 1 to 5 shows a rearing/breeding housing 1 according to a first aspect of the disclosure.

The first aspect of this disclosure shows an insect rearing/breeding housing 1, wherein a plurality of crates 2 arranged in a row along the length of the housing 1 define at least a portion of the housing bottom 3, and wherein holes 4 formed in the bottom of the crates 2 allow for air to be sucked into the housing 1 and to be discharged through at least one air ventilating opening 5 of the housing 1, the housing 1 having a crate insertion opening 6 at one end 7 and a crate discharge opening 8 at an opposite end 9, the crates 2 being supported so as to be movable from the insertion opening 6 to the discharge opening 8.

In an embodiment of the crates 2, holes are provided in sides and/or ends of the crates 2, at least in an upper part of the sides and/or ends of the crates 2. This allows for ventilation both through bottom holes 4 and through sides and/or ends of the crates 2. Hereby is achieved an enhanced ventilation flow through the crates when placed in the housing bottom 3 of the insect rearing/breeding housing 1, and even when the crates are stacked, waiting to be inserted in the insect rearing/breeding housing 1.

In Figure 1, 3 and 5 the rearing/breeding housing 1 shows a row of crates 2 inserted through a crate insertion opening 6 in one end 7 of the housing 1 whereby the crates 2 forms or defines at least a portion of the housing bottom 3.

When starting the rearing or breeding process, the process can advantageously be started by pushing a number of empty crates 2 as well as a number of crates containing pupae into the bottom 3 of the housing 1.

For example, in a housing 1 where the bottom 3 is formed by six crates 2 the first four crates 2 can be empty and the last two crates 2 contains pupae for breeding.

Over the course of three to six days, the pupae develop legs and wings, ultimately emerging as full-grown flies. Within two to three days, female flies are capable of reproduction and to lie eggs, which eggs hatch into larvae and the larvae pupate.

To fit into the breeding process of the insects, the crates are moved in the housing adapting to the process. After a number of days, a further number of crates 2 are inserted in the bottom 3 of the housing 1 whereby an identical number of empty crates are pushed out at the opposite end 9 of the housing 1. After yet a number of days same procedure is followed where after the bottom 3 of the housing 1 is filled with pupae containing crates 2.

Then this sequence continues rearing/breeding the insects and hereby crates 2 where pupae has turned into flies are removed from discharge opening 8 at the backend 9 of the housing 1 in pace with new crates 2 being supplied through the insertion opening 6 at the front end 7 of the housing.

In the bottom 3 of the housing, longitudinal support flanges 10 for supporting the crates 2 is provided at each side 11, 12 of the housing 1 extending from the crate insertion opening 6 to the crate discharge opening 8. The crates 2 can by the edges of the underside of the crates 2 simply support on the flanges 10, or the crates can be formed with an upper edge 16 extending perpendicular from the sides of the crates in a substantially horizontal direction. The edge does not need to be at the upper end of the sides of the crates 2, what is important is that the edge 16 extends from the side of the crate in such a way that the edge 16 can bear on the longitudinal support flanges 10.

In an aspect, a further longitudinal flange is provided at each side 11, 12 of the housing 1, parallel to the support flanges 10 and having a distance from the support flanges 10 corresponding to a height of an edge 16 of the crates 2.

Hereby the crates are prevented from being displaced in a vertical direction when pushed from the inlet opening 6 to the outlet opening 8 and ensures that insects are prevented from escaping the housing 1 through gaps between the edges 16 of the crates 2 and the support flanges 10.

The further longitudinal flanges are preferably placed above the support flanges 10.

In an aspect, ventilating air is supplied through the bottom 3 of the housing 1 below the crates 2. This is possible due to a number of perforations or holes 4 formed in the bottom of the crates 2.

In an aspect, ventilating air is sucked out at top 14 of the housing 1 by a fan or blower (not shown).

In an aspect, ventilating air is supplied through one or more openings 5 at a top part 14 of the housing 1.

In an aspect, ventilating air is alternating between being supplied through one or more openings 5 at a top part 14 of the housing 1 and through the bottom 3 below the crates 2.

To further enhance ventilating flow through the crates 2, a kind of curtains 11a, 12a are provided extending along sides of the crates 2 from the longitudinal support flanges 10 and to at least an underside of the crates 2. This ensures a directional flow of air, which is thereby forced or guided to primarily go up through the holes 4 in bottom of the crates 2 and not partially through the holes in the sides of the crates 2.

The curtains 11a, 12a can be made of metal, plastic or another suitable substantially rigid material that will not flap under the influence of the ventilation air.

In a simple embodiment, the curtains 11a, 12a are formed by the sides 11, 12 of the housing 1, which sides 11, 12 continues from the top 14 of the housing 1 to below or to a point level with the underside of the crates 2 when placed in the housing 1.

In an aspect, one or more ventilating openings 5 at top 14 of the housing 1 are provided with a net or grate (not shown), preventing flies from entering the one or more ventilating openings 5.

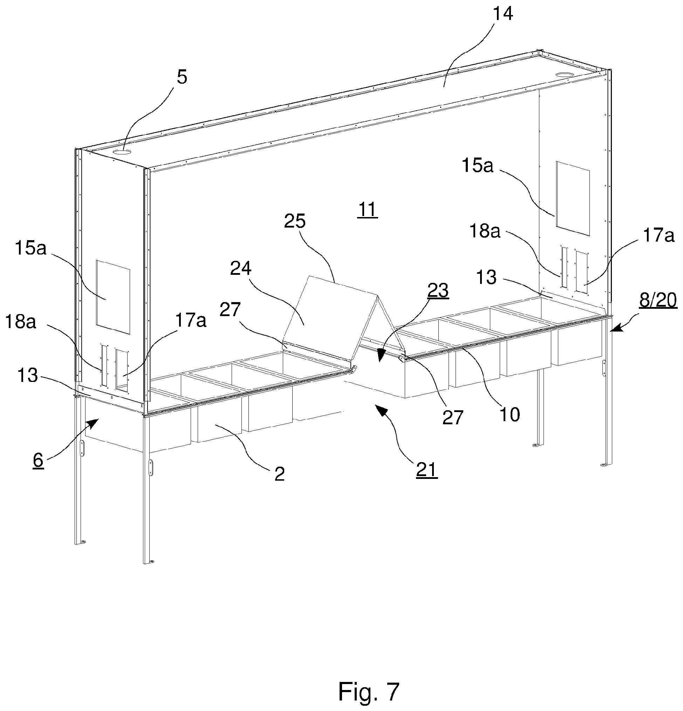

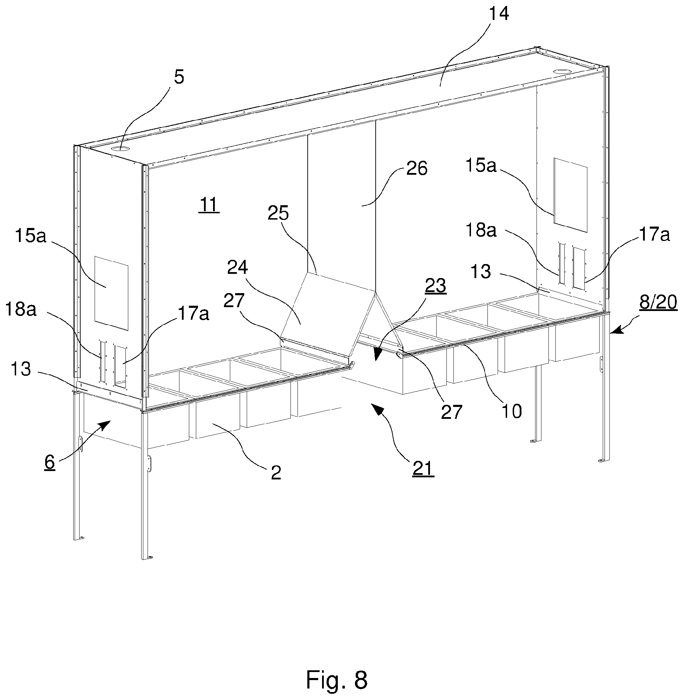

In an aspect, an inspection window 15 is provided at an end 7, 9 of the housing 1. For convenient reasons the inspection window 15 is placed at the end of the housing from where it is operated, normally at the insertion end 7 of the housing 1, but in some embodiments, a further inspection window can be placed at the outlet end 9 or even in both ends 7, 9 as indicated by openings 15a in Figures 7 and 8.

Hereby it is possible to follow if the rearing/breeding process proceeds as planned.

In an aspect, the crate insertion opening 6 at one end 7 and the crate discharge opening 8 at the opposite end 9 of the housing 1 are provided with a sealing 13 for sealing towards an upper edge 16 of the crates 2. This sealing prevents insects from escaping the housing when inserting and removing crates.

The crates 2 are also formed in such a way that the edges on sides of the crates 2 abutting each other, leaving no gaps for insects to escape between the crates 2 forming the bottom 3 of the housing 1.

The egg module 17 is placed on an end 7 of the rearing/breeding housing 1 comprising the insertion opening 6 for the crates 2. The egg module 17 can be placed and replaced from outside the housing and comprises a cardboard hole or cell structure of a type similar to a honeycomb structure of a honey frame for harvesting honey from bees. The egg module 17 also comprises a bait box for emitting a homey smell or lure scent, to attract the flying insects, here female Black Soldier Flies, and encourage the flies to lay eggs in the cells in the egg module 17. When the cells are sufficiently filled with eggs, the eggs are harvested by removing the cardboard structure from the egg module 17 and a new cardboard structure is inserted for further harvesting of eggs. In an embodiment, the whole egg module, including the cardboard structure, can be replaced, when harvesting.

In an embodiment the egg module 17 can be made of other materials, which for example include materials based on plastic or recycled plastic, thereby achieving a greater degree of reusability of egg modules 17 in the housing 1.

Also close to the egg module 17 in the same end 7 of the rearing/breeding housing 1 a water module 18 is placed. The water module 18 provides drinking water to the flies and is configured with a water reservoir 19 at a bottom part of the module, which reservoir 19 extends from outside the housing 1 and into the housing 1. At the side of the water module 18 extending on the inner side of the front end 7 of the housing 1, a porous material due to capillary effect, distributes the water from the reservoir to the surface of the material so that the flies can settle on the material and drink.

The material can be a porous stone or another suitable porous material.

Placing the water module 18 close to the egg module 17 also attracts the flies to the area where you want them to lay their eggs.

The water module can in an embodiment be connected to an external water supply, such as an industrial water supply or a drinking water supply for automatically filling the reservoir, when emptied.

Ventilation of the rearing/breeding housing 1 is necessary due to the fact that insects and especially flies are attracted by strong smells of spoiled things, such as rotten meat or rotten fruits or the like. Hence, the remains from the pupae when turning into flies as well as fly droppings in huge numbers will attract the flies and eventually the smell will drown out the smell from the bait box. Therefore, it is important that the ventilation removes the smell from the crates 2 so that the flies are still attracted to the egg module 17 for laying eggs. Laying eggs in the crates 2 will cause that a huge number of eggs will be extremely difficult to harvest and thereby lost.

To encourage the flies to lay their eggs in the egg module 17, a sufficient level of ventilation ensures, in combination with removal of old crates 2, that a clean environment is maintained within the rearing/breeding housing 1. This ensures that the units can be operated continuously, at least in periods of 2 to 4 weeks, which is cost-efficient.

A sufficient air exchange can be at least 20 times the volume of the rearing/breeding housing 1 per hour. It is also necessary to ensure that the ventilation does not blow remains from pupated pupae around in the housing.

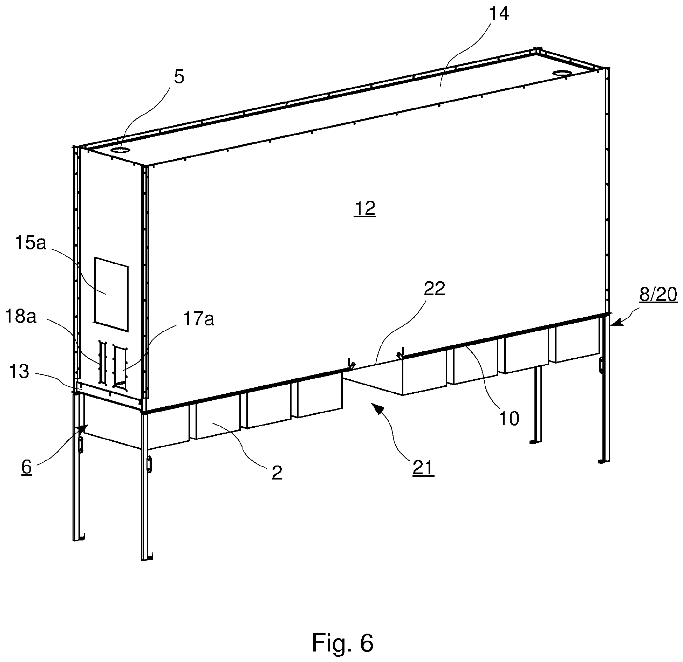

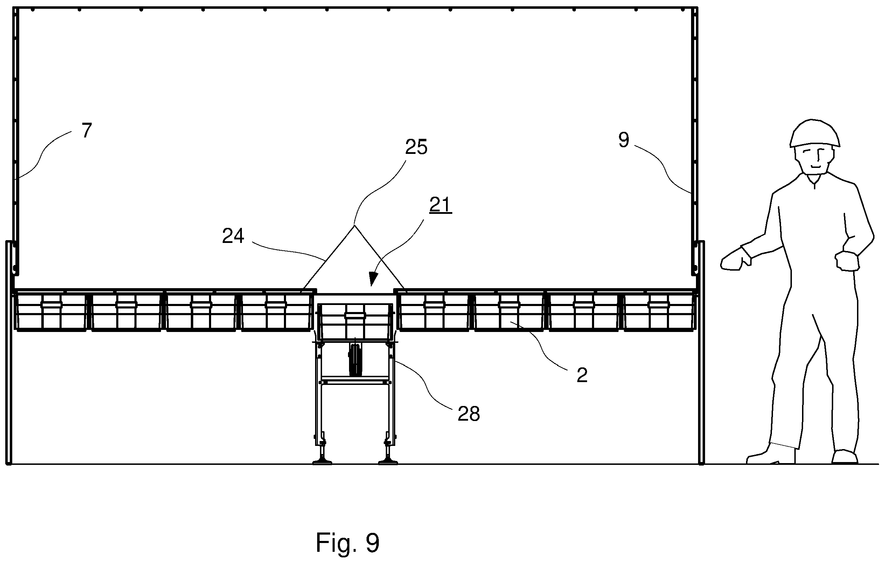

Figure 6 to 10 shows a rearing/breeding housing 1 according to a second aspect of the disclosure.

In embodiments shown in Figures 6 - 10 an egg module 17 and a water module 18 can be provided at each end 7, 9 as indicated by openings 17a and 18a in Figures 7 and 8.

In an aspect, the housing 1 is provided with a crate insertion opening 6, 20 at both ends of said housing 1 and a discharge opening 21 is provided at a middle portion 22 of the housing 1 at the housing bottom 3.

In an aspect, the longitudinal support flanges 10 for supporting the crates 2 provided at each side 11, 12 of the housing 1, extends from the crate insertion openings 6, 20 at each end of the housing 1 to the crate discharge opening 21 provided at a middle portion 22 of the housing 1 at the housing bottom 3.

In an aspect, the longitudinal support flanges 10 leaves a gap 23 at each side 11, 12 of the housing 1, for removal of the crates 2.

In an aspect, a cover plate 24 is provided above and between the gaps 23, which cover plate 24 slopes downwards to at least one side along a moving direction of the crates 2. The sloping sides provides that dead flies or the like will fall into the crates 2 and are thereby removed by the discharged crates 2 to avoid dead flies or the like generating smells attracting flies to lay eggs different places than in the egg module 17.

In an aspect, a separating wall or plate 26 extends from a top part 25 of the cover plate 24 to the top 14 of the housing 1, separating the housing 1 in two.

Separating the housing 1 in two makes it possible for rearing/breeding in two different enclosures in the same housing 1, and thereby keeping the populations separated from each other.

In an aspect, the cover plate 24 is provided with a sealing 27 for sealing towards an upper edge 16 of the crates 2.

In an aspect, the cover plate 24 is provided with a sealing 27 at each side for sealing towards an upper edge 16 of the crates 2 to prevent insects from escaping the housing 1 when discharging crates 2 from the discharge opening 8, 21.

In an aspect, a conveyor 28 is provided at the discharge opening 8, 21 for conveying discharged crates 2 in a crosswise direction in relation to movement of the crates 2 in the housing 1.

In an aspect, the conveyor 28 is placed below the crate discharge opening 21 at a middle portion 22 of the housing 1 at the housing bottom 3.

Providing a conveyor 28 to handle discharged crates 2 from the housing 1 gives possibilities for building of a more flexible rearing/breeding system 29 comprising a plurality of rearing/breeding housings 1 placed next to each other.

The conveyor 28 can be placed on a supporting frame or another kind of known supports for conveyors 28.

In the aspects where the longitudinal support flanges 10 leaves a gap 23 at each side 11, 12 of the housing 1, for removal of the crates 2, a similar gap in curtains 11a, 12a, provided extending along sides of the crates 2 from the longitudinal support flanges 10 and to at least an underside of the crates 2 may be provided to let the crates 2 be transported on the conveyor 28.

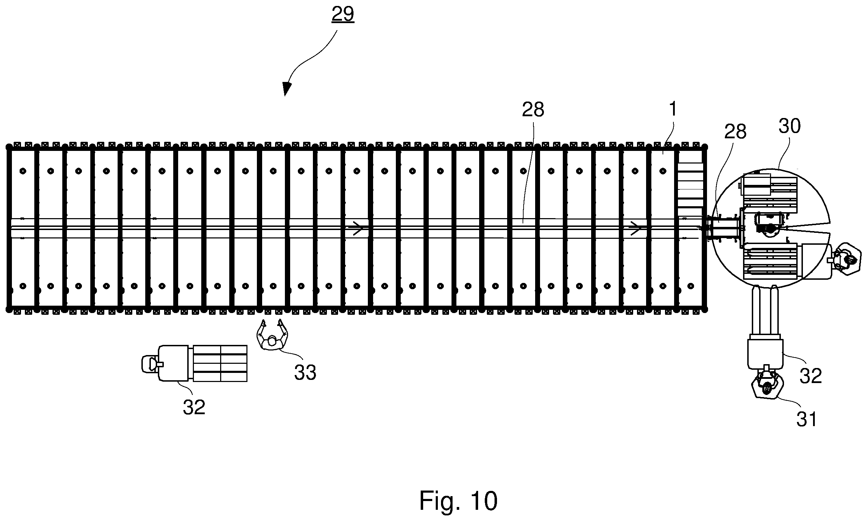

Figure 10 shows a rearing/breeding system 29 comprising a plurality of rearing/breeding housings 1 placed next to each other and with a conveyor 28 placed in a crosswise direction in relation to a moving direction of the crates 2 in the housings 1.

At one end of the conveyor 28 from where crates 2 form the housings are discharged a handling area is provided where an operator 31 can remove discharges crates 2 by means of a pallet lifter, a forklift or similar transport equipment 32.

An operator 33 for harvesting eggs from the egg modules, operates along the ends 7, 9 of the plurality of housings 1 and can place the harvested eggs on pallets, crates, baskets or the like and transport them by means of a pallet lifter, a forklift or similar transport equipment 32.

In an aspect one or more of the operators 31, 33 can be assisted or replaced by a robot or other automated equipment for handling the egg modules 17 or crates 2.

In an aspect the housings 1 are placed on supporting legs or frames for supporting the housings 1 in a distance above a floor or above the ground in a building or similar.

In another aspect, the housings 1 are supported by support means (not shown), such as wires, stays, rods or the like, which hang down from a ceiling or other support structure situated above the housings 1, such that the housings 1 hang freely over a floor or similar. This makes it easier to clean under the houses.

The person skilled in the art realizes that the present disclosure is not limited to the preferred embodiments described above. The person skilled in the art further realizes that modifications and variations are possible within the scope of the appended claims. For example, egg modules and water modules can be placed different places what is important is that it is possible for an operator to exchange the modules, refill water and harvest the eggs. Additionally, variations to the disclosed embodiments can be understood and effected by the skilled person in practicing the claimed disclosure, from a study of the drawings, the disclosure, and the appended claims.

## Claims

1. An insect rearing/breeding housing (1), the housing defined by one end (7) and an opposite end (9), sides (11,12) and a top (14), wherein a plurality of crates (2) having a bottom and an upper edge (16) and arranged in a row along the length of the housing (1) define at least a portion of the housing bottom (3), where the housing bottom (3) is closed by crates and wherein holes (4) formed in the bottom of the crates (2) allow for air to be sucked into the housing (1) and to be discharged through at least one air ventilating opening (5) of the housing (1), the housing (1) having a crate insertion opening (6, 20) in at least one of the ends (7, 9) and a crate discharge opening (8, 21) at the housing bottom (3), said crates (2) being supported so as to be movable from said insertion opening (6, 20) to said discharge opening (8, 21).

2. The insect rearing/breeding housing (1) according to claim 1, wherein the crate discharge opening (21) is provided at the opposite end (9) in relation to the crate insertion opening (6) in one end (7) and at the housing bottom (3).

3. The insect rearing/breeding housing according to claim 1, wherein the housing (1) is provided with a crate insertion opening (6, 8) at both ends (7, 9) of said housing (1) and a discharge opening (21) is provided at a middle portion (22) of the housing (1) at the housing bottom (3).

4. The insect rearing/breeding housing according to claim 2 or 3, wherein longitudinal support flanges (10) for supporting the crates (2) are provided at each side (11, 12) of the housing (1) extending from a crate insertion opening (6, 20) to a crate discharge opening (8, 21).

5. The insect rearing/breeding housing (1) according to claim 4, wherein a longitudinal flange is provided at each side (11, 12) of the housing (1), parallel to the support flanges (10) and having a distance from the support flanges (10) corresponding to a height of an edge of the crates (2).

6. The insect rearing/breeding housing according to claim 3, wherein the longitudinal support flanges (10) for supporting the crates (2) provided at each side (11, 12) of the housing (1), extends from the crate insertion openings (6, 20) at each end of the housing (1) to the crate discharge opening (21) provided at a middle portion (22) of the housing (1) at the housing bottom (3).

7. The insect rearing/breeding housing according to claim 6, wherein the longitudinal support flanges (10) leaves a gap (23) at each side (11, 12) of the housing (1), for removal of the crates (2).

8. The insect rearing/breeding housing according to claim 7, wherein a cover plate (24) is provided above and between the gaps (23), which cover plate (24) slopes downwards to at least one side along a moving direction of the crates (2).

9. The insect rearing/breeding housing according to claim 8, wherein a separating wall or plate (26) extends from a top part (25) of the cover plate (24) to the top (14) of the housing (1), separating the housing (1) in two.

10. The insect rearing/breeding housing according to claim 8 or 9, wherein the cover plate (24) is provided with a sealing (27) for sealing towards the upper edge (16) of the crates (2).

11. The insect rearing/breeding housing (1) according to any one or more of the claims 1 - 10, wherein the housing (1) comprises a fan or blower configured to suck the ventilated air out at the top (14) of the housing (1).

12. The insect rearing/breeding housing according to any one or more of the claims 1 - 10, wherein the housing (1) is configured for supply of ventilating air, alternating between being supplied through one or more openings (5) at a top part (14) of the housing (1) and through the bottom (3) from below the crates (2).

13. The insect rearing/breeding housing according to any one or more of the claims 1 - 12, wherein a kind of curtains 11a, 12a are provided extending along sides of the crates 2 from the longitudinal support flanges 10 and to at least an underside of the crates 2.

14. The insect rearing/breeding housing (1) according to any one or more of the claims 1 - 13 wherein one or more ventilating openings (5) at the top (14) of the housing (1) are provided with a net or grate, preventing flies from entering the one or more ventilating openings (5).

15. The insect rearing/breeding housing (1) according to any one or more of the claims 1 - 14 wherein an inspection window (15) is provided at an end (7, 9) of the housing (1).

16. The insect rearing/breeding housing according to any one or more of the claims 1 - 2, wherein the crate insertion opening (6, 20) and/or crate discharge opening (8) is provided with a sealing (13) for sealing towards the upper edge (16) of the crates (2).

17. An insect rearing/breeding system (29) comprising a plurality of rearing/breeding housings (1) according to any of the preceding claims, the rearing/breeding housings (1) placed next to each other, the system further comprising a conveyor (28) placed in a crosswise direction in relation to a moving direction of the crates (2) in the housings (1).

18. The insect rearing/breeding system according to claim 17, wherein the conveyor (28) is placed below the crate discharge opening (21) at a middle portion (22) of the housing (1) at the housing bottom (3).

## Patentansprüche

1. Insektenzucht-/-anzuchtgehäuse (1), wobei das Gehäuse durch ein Ende (7) und ein gegenüberliegendes Ende (9), Seiten (11, 12) und eine Oberkante (14) definiert ist, wobei eine Vielzahl von Kisten (2), die eine Unterkante und einen oberen Rand (16) aufweist und in einer Reihe entlang der Länge des Gehäuses (1) angeordnet ist, mindestens einen Abschnitt der Gehäuseunterkante (3) definiert, wobei die Gehäuseunterkante (3) durch Kisten verschlossen ist und wobei Löcher (4), die in der Unterkante der Kisten (2) gebildet sind, es ermöglichen, dass Luft in das Gehäuse (1) hineingesogen und durch mindestens eine Luftventilationsöffnung (5) des Gehäuses (1) ausgegeben wird, wobei das Gehäuse (1) eine Kisteneinführungsöffnung (6, 20) in mindestens einem der Enden (7, 9) und eine Kistenausgabeöffnung (8, 21) an der Gehäuseunterkante (3) aufweist, wobei die Kisten (2) gestützt werden, um von der Einführungsöffnung (6, 20) zu der Ausgabeöffnung (8, 21) bewegbar zu sein.

2. Insektenzucht-/-anzuchtgehäuse (1) nach Anspruch 1, wobei die Kistenausgabeöffnung (21) an dem gegenüberliegenden Ende (9) in Bezug auf die Kisteneinführungsöffnung (6) in einem Ende (7) und an der Gehäuseunterkante (3) bereitgestellt ist.

3. Insektenzucht-/-anzuchtgehäuse nach Anspruch 1, wobei das Gehäuse (1) mit einer Kisteneinführungsöffnung (6, 8) an beiden Enden (7, 9) des Gehäuses (1) versehen ist und eine Ausgabeöffnung (21) an einem mittleren Abschnitt (22) des Gehäuses (1) an der Gehäuseunterkante (3) bereitgestellt ist.

4. Insektenzucht-/-anzuchtgehäuse nach Anspruch 2 oder 3, wobei Längsstützflansche (10) zum Stützen der Kisten (2) an jeder Seite (11, 12) des Gehäuses (1) bereitgestellt sind, die sich von einer Kisteneinführungsöffnung (6, 20) zu einer Kistenausgabeöffnung (8, 21) erstrecken.

5. Insektenzucht-/-anzuchtgehäuse (1) nach Anspruch 4, wobei ein Längsflansch an jeder Seite (11, 12) des Gehäuses (1) bereitgestellt ist, der parallel zu den Stützflanschen (10) verläuft und eine Distanz von den Stützflanschen (10) aufweist, die einer Höhe eines Randes der Kisten (2) entspricht.

6. Insektenzucht-/-anzuchtgehäuse nach Anspruch 3, wobei sich die Längsstützflansche (10) zum Stützen der Kisten (2), die an jeder Seite (11, 12) des Gehäuses (1) bereitgestellt sind, von den Kisteneinführungsöffnungen (6, 20) an jedem Ende des Gehäuses (1) zu der Kistenausgabeöffnung (21) erstrecken, die an einem mittleren Abschnitt (22) des Gehäuses (1) an der Gehäuseunterkante (3) bereitgestellt ist.

7. Insektenzucht-/-anzuchtgehäuse nach Anspruch 6, wobei die Längsstützflansche (10) an jeder Seite (11, 12) des Gehäuses (1) eine Lücke (23) zur Entfernung der Kisten (2) lassen.

8. Insektenzucht-/-anzuchtgehäuse nach Anspruch 7, wobei eine Abdeckplatte (24) über und zwischen den Lücken (23) bereitgestellt ist, wobei die Abdeckplatte (24) zu mindestens einer Seite entlang einer Bewegungsrichtung der Kisten (2) nach unten neigt.

9. Insektenzucht-/-anzuchtgehäuse nach Anspruch 8, wobei sich eine Trennwand oder -platte (26) von einem Oberkantenteil (25) der Abdeckplatte (24) zu der Oberkante (14) des Gehäuses (1) erstreckt und das Gehäuse (1) zweiteilt.

10. Insektenzucht-/-anzuchtgehäuse nach Anspruch 8 oder 9, wobei die Abdeckplatte (24) mit einer Abdichtung (27) zum Abdichten in Richtung des oberen Randes (16) der Kisten (2) versehen ist.

11. Insektenzucht-/-anzuchtgehäuse (1) nach einem oder mehreren der Ansprüche 1-10, wobei das Gehäuse (1) einen Ventilator oder ein Gebläse umfasst, der/das dazu konfiguriert ist, die ventilierte Luft an der Oberkante (14) des Gehäuses (1) hinauszusaugen.

12. Insektenzucht-/-anzuchtgehäuse nach einem oder mehreren der Ansprüche 1-10, wobei das Gehäuse (1) zur Zufuhr ventilierter Luft, abwechselnd zwischen Zuführen durch eine oder mehrere Öffnungen (5) an einem Oberkantenteil (14) des Gehäuses (1) und durch die Unterkante (3) von unterhalb der Kisten (2), konfiguriert ist.

13. Insektenzucht-/-anzuchtgehäuse nach einem oder mehreren der Ansprüche 1-12, wobei eine Art Vorhänge 11a, 12a bereitgestellt sind, die sich entlang Seiten der Kisten 2 von den Längsstützflanschen 10 und zu mindestens einer Unterseite der Kisten 2 erstrecken.

14. Insektenzucht-/-anzuchtgehäuse (1) nach einem oder mehreren der Ansprüche 1-13, wobei eine oder mehrere Ventilationsöffnungen (5) an der Oberkante (14) des Gehäuses (1) mit einem Netz oder Gitter versehen sind, das Fliegen daran hindert, in die eine oder die mehreren Ventilationsöffnungen (5) hineinzugelangen.

15. Insektenzucht-/-anzuchtgehäuse (1) nach einem oder mehreren der Ansprüche 1-14, wobei ein Prüffenster (15) an einem Ende (7, 9) des Gehäuses (1) bereitgestellt ist.

16. Insektenzucht-/-anzuchtgehäuse nach einem oder mehreren der Ansprüche 1-2, wobei die Kisteneinführungsöffnung (6, 20) und/oder die Kistenausgabeöffnung (8) mit einer Abdichtung (13) zum Abdichten in Richtung des oberen Randes (16) der Kisten (2) versehen ist.

17. Insektenzucht-/-anzuchtsystem (29), umfassend eine Vielzahl von Zucht-/Anzuchtgehäusen (1) nach einem der vorhergehenden Ansprüche, wobei die Zucht-/Anzuchtgehäuse (1) nebeneinander platziert sind, wobei das System ferner ein Förderband (28) umfasst, das in einer Querrichtung in Bezug auf eine Bewegungsrichtung der Kisten (2) in den Gehäusen (1) platziert ist.

18. Insektenzucht-/-anzuchtsystem nach Anspruch 17, wobei das Förderband (28) unterhalb der Kistenausgabeöffnung (21) an einem mittleren Abschnitt (22) des Gehäuses (1) an der Gehäuseunterkante (3) platziert ist.

## Revendications

1. Logement d'élevage/de reproduction d'insectes (1), le logement étant défini par une extrémité (7) et une extrémité opposée (9), des côtés (11, 12) et une partie supérieure (14), dans lequel une pluralité de caisses (2) possédant un fond et un bord supérieur (16) et agencées en une rangée le long de la longueur du logement (1) définissent au moins une partie du fond de logement (3), où le fond de logement (3) est fermé par des caisses et dans lequel des trous (4) formés dans le fond des caisses (2) permettent à l'air d'être aspiré dans le logement (1) et d'être évacué à travers au moins une ouverture de ventilation d'air (5) du logement (1), le logement (1) possédant une ouverture d'insertion de caisse (6, 20) dans au moins l'une des extrémités (7, 9) et une ouverture de déchargement de caisse (8, 21) au niveau du fond de logement (3), lesdites caisses (2) étant supportées de manière à être mobiles de ladite ouverture d'insertion (6, 20) vers ladite ouverture de déchargement (8, 21).

2. Logement d'élevage/de reproduction d'insectes (1) selon la revendication 1, dans lequel l'ouverture de déchargement de caisse (21) est prévue à l'extrémité opposée (9) par rapport à l'ouverture d'insertion de caisse (6) dans une extrémité (7) et au niveau du fond de logement (3).

3. Logement d'élevage/de reproduction d'insectes selon la revendication 1, dans lequel le logement (1) est doté d'une ouverture d'insertion de caisse (6, 8) aux deux extrémités (7, 9) dudit logement (1) et une ouverture de déchargement (21) est prévue au niveau d'une partie centrale (22) du logement (1) au niveau du fond de logement (3).

4. Logement d'élevage/de reproduction d'insectes selon la revendication 2 ou 3, dans lequel des brides de support longitudinales (10) pour supporter les caisses (2) sont prévues de chaque côté (11, 12) du logement (1) s'étendant d'une ouverture d'insertion de caisse (6, 20) vers une ouverture de déchargement de caisse (8, 21).

5. Logement d'élevage/de reproduction d'insectes (1) selon la revendication 4, dans lequel une bride longitudinale est prévue de chaque côté (11, 12) du logement (1), parallèlement aux brides de support (10) et présentant une distance par rapport aux brides de support (10) correspondant à une hauteur d'un bord des caisses (2).

6. Logement d'élevage/de reproduction d'insectes selon la revendication 3, dans lequel les brides de support longitudinales (10) pour supporter les caisses (2) prévues de chaque côté (11, 12) du logement (1), s'étendent depuis les ouvertures d'insertion de caisse (6, 20) à chaque extrémité du logement (1) vers l'ouverture de déchargement de caisse (21) prévue au niveau d'une partie centrale (22) du logement (1) au niveau du fond de logement (3).

7. Logement d'élevage/de reproduction d'insectes selon la revendication 6, dans lequel les brides de support longitudinales (10) laissent un espace (23) de chaque côté (11, 12) du logement (1), pour le retrait des caisses (2).

8. Logement d'élevage/de reproduction d'insectes selon la revendication 7, dans lequel une plaque de couverture (24) est prévue au-dessus et entre les espaces (23), laquelle plaque de couverture (24) s'incline vers le bas vers au moins un côté le long d'une direction de déplacement des caisses (2).

9. Logement d'élevage/de reproduction d'insectes selon la revendication 8, dans lequel une paroi ou plaque de séparation (26) s'étend d'une partie supérieure (25) de la plaque de couverture (24) vers la partie supérieure (14) du logement (1), séparant le logement (1) en deux.

10. Logement d'élevage/de reproduction d'insectes selon la revendication 8 ou 9, dans lequel la plaque de couverture (24) est dotée d'un joint (27) pour assurer l'étanchéité vers le bord supérieur (16) des caisses (2).

11. Logement d'élevage/de reproduction d'insectes (1) selon l'une quelconque ou plusieurs des revendications 1 à 10, dans lequel le logement (1) comprend un ventilateur ou une soufflante configurée pour réaliser l'aspiration de l'air ventilé vers l'extérieur au niveau de la partie supérieure (14) du logement (1).

12. Logement d'élevage/de reproduction d'insectes selon l'une quelconque ou plusieurs des revendications 1 à 10, dans lequel le logement (1) est configuré pour l'alimentation en air de ventilation, alternant entre une alimentation à travers une ou plusieurs ouvertures (5) au niveau d'une partie supérieure (14) du logement (1) et à travers le fond (3) depuis le dessous des caisses (2).

13. Logement d'élevage/de reproduction d'insectes selon l'une quelconque ou plusieurs des revendications 1 à 12, dans lequel une sorte de rideaux 11a, 12a sont prévus s'étendant le long des côtés des caisses 2 depuis les brides de support longitudinales 10 et jusqu'à au moins un dessous des caisses 2.

14. Logement d'élevage/de reproduction d'insectes (1) selon l'une quelconque ou plusieurs des revendications 1 à 13, dans lequel les une ou plusieurs ouvertures de ventilation (5) au niveau de la partie supérieure (14) du logement (1) sont dotées d'un filet ou d'une grille, empêchant les mouches de pénétrer dans les une ou plusieurs ouvertures de ventilation (5).

15. Logement d'élevage/de reproduction d'insectes (1) selon l'une quelconque ou plusieurs des revendications 1 à 14, dans lequel une fenêtre d'inspection (15) est prévue à une extrémité (7, 9) du logement (1).

16. Logement d'élevage/de reproduction d'insectes selon l'une quelconque ou plusieurs des revendications 1 à 2, dans lequel l'ouverture d'insertion de caisse (6, 20) et/ou l'ouverture de déchargement de caisse (8) est dotée d'un joint (13) pour assurer l'étanchéité vers le bord supérieur (16) des caisses (2).

17. Système d'élevage/de reproduction d'insectes (29) comprenant une pluralité de logements d'élevage/de reproduction (1) selon l'une quelconque des revendications précédentes, les logements d'élevage/de reproduction (1) étant placés les uns à côté des autres, le système comprenant en outre un convoyeur (28) placé dans une direction transversale par rapport à une direction de déplacement des caisses (2) dans les logements (1).

18. Système d'élevage/de reproduction d'insectes selon la revendication 17, dans lequel le convoyeur (28) est placé sous l'ouverture de déchargement de caisse (21) au niveau d'une partie centrale (22) du logement (1) au niveau du fond de logement (3).
